# EUROPEAN PATENT SPECIFICATION

(11) **EP 4 195 941 B1**
(45) Date of publication and mention of the grant of the patent: **07.10.2026**
(21) Application number: 21856830.1
(22) Date of filing: 13.08.2021
(51) Int. Cl.: A61K 9/48, A23L 2/52, A23L 33/105, A61K 9/107, A61K 31/045, A61K 31/05, A61K 31/352, A61K 36/185, A61K 36/67, A61K 45/06, A61K 47/24, A61K 9/06, A61K 31/015

(54) **IMMUNOMODULATION FORMULATIONS AND RELATED METHODS**
IMMUNMODULATIONSFORMULIERUNGEN UND ZUGEHÖRIGE VERFAHREN
FORMULATIONS D'IMMUNOMODULATION ET PROCÉDÉS ASSOCIÉS

(30) Priority: 13.08.2020 US 202063065301 P
(43) Date of publication of application: 21.06.2023
(73) Proprietor: Vickers, Alexander, Addison, TX 75001 (US)
(72) Inventor: Vickers, Alexander, Addison, TX 75001 (US)
(74) Representative: Barker Brettell LLP
(86) International application number: PCT/US2021/046032
(87) International publication number: WO 2022/036278

(56) References cited:
- EP-A1- 3 409 275
- EP-A1- 3 417 846
- WO-A1-2019/104291
- WO-A1-2019/135224
- WO-A1-2019/135224
- WO-A1-2019/136351
- WO-A1-2021/033149
- US-A1- 2002 086 042
- US-A1- 2011 195 096
- US-A1- 2016 051 510
- BO WANG ET AL: "In Search of Preventative Strategies: Novel Anti-Inflammatory High-CBD <em>Cannabis Sativa</em> Extracts Modulate ACE2 Expression in COVID-19 Gateway Tissues", 17 April 2020 (2020-04-17), XP055765023, Retrieved from the Internet <URL:https://pubmed.ncbi.nlm.nih.gov/33221759/> DOI: 10.20944/preprints202004.0315.v1
- ROSSI FRANCESCA ET AL: "Cannabinoid Receptor Type 2: A Possible Target in SARS-CoV-2 (CoV-19) Infection?", INTERNATIONAL JOURNAL OF MOLECULAR SCIENCES, vol. 21, no. 11, 27 May 2020 (2020-05-27), pages 1 - 16, XP055879942, DOI: 10.3390/ijms21113809
- GIUSEPPE ESPOSITO ET AL: "The potential of cannabidiol in the COVID-19 pandemic", BRITISH JOURNAL OF PHARMACOLOGY, WILEY-BLACKWELL, UK, vol. 177, no. 21, 16 July 2020 (2020-07-16), pages 4967 - 4970, XP071172311, ISSN: 0007-1188, DOI: 10.1111/BPH.15157
- BO WANG, ANNA KOVALCHUK, DONGPING LI, YAROSLAV ILNYTSKYY, IGOR KOVALCHUK, OLGA KOVALCHUK: "In Search of Preventative Strategies: Novel Anti-Inflammatory High-CBD <em>Cannabis Sativa</em> Extracts Modulate ACE2 Expression in COVID-19 Gateway Tissues", XP055765023, Retrieved from the Internet <URL:<fi!e://core-fs-01/FTEHome$/CS01/Downloads/preprints202004.0315.v1.pdf> [retrieved on 20211028], DOI: 10.20944/preprints202004.0315.v1
- GIUSEPPE ESPOSITO; MARCELLA PESCE; LUISA SEGUELLA; WALTER SANSEVERINO; JIE LU; CHIARA CORPETTI; GIOVANNI SARNELLI: "The potential of cannabidiol in the COVID‐19 pandemic", BRITISH JOURNAL OF PHARMACOLOGY, vol. 177, no. 21, 16 July 2020 (2020-07-16), UK , pages 4967 - 4970, XP071172311, ISSN: 0007-1188, DOI: 10.1111/bph.15157

## Description

### TECHNICAL FIELD

The present disclosure relates to methods and compositions for treating and/or preventing a disease or disorder caused by a coronavirus infection.

### BACKGROUND

SARS-CoV-2 (COVID-19) is a sense RNA virus with envelope- and spike-like projections on its surface. Coronaviruses can infect a wide range of vertebrates, including humans. Coronaviruses can manifest with a variety of symptoms from mild to severe (e.g., flu, fever, cough, fatigue, shortness of breath, infection of the lower respiratory tract, pneumonia, fibrosis with thrombosis in pulmonary small vessels, etc.) and even death. Coronaviruses can also lead to complications associated with the immune response being out of control, such as disseminated intravascular coagulation (DIC). The severity of the disease may depend on the efficiency of the immune system of affected individuals and the presence of co-morbidities. A common feature is the strong inflammatory response, which manifests through elevated C-reactive protein (CRP), pro-inflammatory cytokines production (Il-6, IL-10, IL-1), higher TNF-α, neutrophil count, D-dimer and blood urea. SARS-CoV-2 spreads in the population at a rate of 0.8%-3%, more than the normal flu and binds to angiotensin-converting enzyme 2 (ACE2) with high affinity to infect humans.

Patent application EP3417846A1 discloses a food and/or nutraceutical composition, for example in the form of liposomes, which comprises an endocannabinoid, an active ingredient and an excipient.

Patent application WO2019135224A1 discloses a stable oil-in-water submicron emulsion oral cannabinoid composition, comprising at least one cannabinoid in a pharmaceutically acceptable carrier, at least one triglyceride oil, at least two emulsifiers, at least one taste-enhancing excipient and water, and related methods and kits.

Patent application US2016051510A1 discloses films (ODFs) that include botanical extract, and related methods.

Patent application EP3409275A1 discloses a composition comprising palmitoylethanolamide, curcumin or a plant extract belonging to genus *Curcuma* comprising curcumin, and optionally piperine or a plant extract belonging to the *Piperaceae* family comprising piperine, and related uses.

The publication Wang et al. "In Search of Preventative Strategies: Novel Anti-Inflammatory High-CBD Cannabis Sativa Extracts Modulate ACE2 Expression in COVID-19 Gateway Tissues" Europe PMC (2020): PPR152713 (doi: 10.20944/preprints202004.0315.v1) discloses high CBD C. *sativa* extracts that modulate ACE2 gene expression and ACE2 protein levels.

The publication Esposito et al. "The potential of cannabidiol in the COVID-19 pandemic" British journal of pharmacology 177:21 (2020): 4967-4970 (doi: 10.1111/bph.15157) discloses the hypothesis that cannabidiol (CBD) has the potential to limit the severity and progression of COVID-19.

The publication Rossi et al. "Cannabinoid receptor type 2: a possible target in SARS-CoV-2 (CoV-19) infection?" International Journal of Molecular Sciences 21:11 (2020): 3809 (doi: 10.3390/ijms21113809) discloses the hypothesis that that CB2 receptor can be a therapeutic target in COVID-19.

### SUMMARY

According to a first aspect of the present invention, there is provided a composition for the use according to claim 1 herein. According to a second aspect of the present invention, there is provided a method of preparing a composition, according to claim 9 herein.

The disclosed principles provide for a composition for immunomodulation against coronaviruses, a method of manufacturing the composition, and method of immunomodulation to combat and treat coronaviruses, as well as the symptoms found in humans resulting from infection by coronaviruses.

**In** one embodiment, the composition includes an emulsion formed from a pharmaceutically acceptable carrier mixed with active ingredients. The pharmaceutically acceptable carrier is between 15-85 wt% of the composition. The active ingredients include an effective amount of a hemp extract to provide a source of exogenous cannabinoids, an effective amount of a cannabinoid enhancer to inhibit cannabinoid hydrolases, an effective amount of a fatty acid amide to enhance cannabinoid activity via an entourage effect, an effective amount of a kava extract to alleviate anxiety, and an effective amount of an alkaloid to enhance bioavailability of one or more of the active ingredients. As used herein, the term "effective amount" means a sufficient amount of a compound that can significantly induce a positive modification the condition being treated, but low enough to avoid unwanted side effects, within the scope of sound judgment of a skilled artisan. The effective amount of a compound may vary with the particular condition being treated, the age and condition of the biological subject being treated, the severity of the condition, the duration of the treatment, and other factors within the knowledge and expertise of the skilled artisan.

In another embodiment, the method of manufacturing the composition may comprise the step of combining a pharmaceutically acceptable carrier with active ingredients to form a solution. The active ingredients include an effective amount of a hemp extract to provide a source of exogenous cannabinoids, an effective amount of a cannabinoid enhancer to inhibit cannabinoid hydrolases, an effective amount of a fatty acid amide to enhance cannabinoid activity via an entourage effect, and an effective amount of an alkaloid to enhance bioavailability of one or more of the active ingredients. The method also includes the steps of cooling the solution to a temperature less than about 60°C, adding a kava extract to the cooled solution, and further cooling the cooled solution to a temperature less than about 0°C to form the composition.

### BRIEF DESCRIPTION OF THE DRAWINGS

For a more complete understanding of the present disclosure and its advantages, reference is now made to the following description and the accompanying drawings, in which:
**FIGURE 1** illustrates a flowchart of a method for forming an immunomodulating composition in accordance with an illustrative embodiment;
**FIGURE 2** illustrates a flowchart of a method for combining a pharmaceutically acceptable carrier with active ingredients to form a solution according to an illustrative embodiment; and
**FIGURE 3** illustrates a flowchart of a method for treating an illness using an immunomodulating composition according to an illustrative embodiment.

### DETAILED DESCRIPTION

The following detailed description includes exemplary embodiments of the disclosure and reference is made to the accompanying figures that form a part hereof. The figures here are shown to illustrate embodiments in which the disclosed principles may be practiced. Other embodiments will be utilized which may include structural changes and modifications made without departing from the scope of the present disclosure.

Presently, supporting treatments of COVID-19 focus on the side effects caused by the virus such as inflammation and pulmonary fibrosis (recognized as the first causes of death), and symptomatic and respiratory support (oxygen therapy and extracorporeal membrane oxygenation). In some circumstances, convalescent plasma and immunoglobulin G have been administered to patients. Antiviral drugs and systemic corticosteroid treatment that are commonly used against influenza viruses are inefficient to treat COVID-19. Vaccines that have been developed to protect individuals against the virus are not completely effective at preventing infection. Moreover, some vaccines have proven to have a reduced effect against mutations of the COVID-19 virus.

One of the reasons the SARS-CoV-2 virus is so 'successful' - and thus dangerous - is that it can suppress this non-specific immune response. In addition, it lets the human cell produce the viral protein PLpro (papain-like protease). PLpro has two functions: It plays a role in the maturation and release of new viral particles, and it suppresses the development of type 1 interferons. Interferons (IFNs) are a group of signaling proteins made by host cells and released in response to the presence of several viruses. IFNs belong to the large class of proteins known as cytokines, molecules used for communication between cells to trigger the protective defenses of the immune system that help eradicate pathogens. Interferons are named for their ability to "interfere" with viral replication by protecting cells from virus infections. IFNs also have various other functions: they activate immune cells, such as natural killer cells and macrophages; they increase host defenses by up-regulating antigen presentation by virtue of increasing the expression of major histocompatibility complex (MHC) antigens. Certain symptoms of infections, such as fever, muscle pain and "flu-like symptoms", are also caused by the production of IFNs and other cytokines.

Cannabinoids can downregulate cytokine and chemokine production and, in some models, upregulate T-regulatory cells to suppress inflammatory responses. The endocannabinoid system is also involved in immunoregulation. For example, administration of endocannabinoids or use of inhibitors of enzymes that break down the endocannabinoids led to immunosuppression and recovery from immune-mediated injury to organs such as the liver. Manipulation of endocannabinoids and/or use of exogenous cannabinoids *in vivo* can constitute a potent treatment modality against inflammatory disorders.

The compounds disclosed herein may be utilized as a multi-receptor method of immunomodulation (immune capacity booster) to combat and treat coronaviruses (e.g., COVID-19) as well as the possibility of other autoimmune related diseases including Multiple Sclerosis (MS), Lyme disease, and lymphoma. The disclosed preparation of the ingredients of the disclosed compounds may synergize to "immunomodulate" or regulate/enhance immune function, and also reduce hypertension. The disclosed compounds may include components (i.e., ingredients), which have been shown to modulate ACE 2 expression in tissues where the SARS CoV-2 virus enters the body and also down regulate TMPRSS2, which the virus uses for S protein priming at the ACE 2 site (Hoffmann et al., 2020). The disclosed components may stimulate the release of type 1 interferon, and combat PlPro, a substance created by the virus, which produces and releases new viruses and suppresses the development of type 1 interferon (Hoffmann et al., 2020). The disclosed compounds may target, with CB₂ agonists, the classical cannabinoid receptor type 2 or CB₂ receptor to release Interferon.

ACE2, which stands for angiotensin-converting enzyme 2, is a protein that sits on the surface of many types of cells in the human body. ACE2 receptors mediate the entry into the cell of three strains of coronavirus: SARS-CoV, NL63 and SARS-CoV-2. ACE2 receptors are ubiquitous and widely expressed in the heart, vessels, gut, lung (particularly in type 2 pneumocytes and macrophages), kidney, testis and brain. ACE2 is mostly bound to cell membranes and only scarcely present in the circulation in a soluble form. An important salutary function of membrane-bound and soluble ACE2 is the degradation of angiotensin II to angiotensin1-7. Consequently, ACE2 receptors limit several detrimental effects resulting from binding of angiotensin II to AT1 receptors, which include vasoconstriction, enhanced inflammation and thrombosis. The increased generation of angiotensin1-7 also triggers counter-regulatory protective effects through binding to G-protein coupled Mas receptors.

Unfortunately, the entry of SARS-CoV2 into the cells through membrane fusion markedly down-regulates ACE2 receptors, with loss of the catalytic effect of these receptors at the external site of the membrane. Increased pulmonary inflammation and coagulation have been reported as unwanted effects of enhanced and unopposed angiotensin II effects via the ACE→Angiotensin II→AT1 receptor axis. Patients infected with SARS-CoV-2 show that several features associated with infection and severity of the disease (e.g., older age, hypertension, diabetes, cardiovascular disease) share a variable degree of ACE2 deficiency. Concerning the disclosed compounds, ACE2 down-regulation induced by viral invasion may be detrimental in people with baseline ACE2 deficiency associated with the above conditions. The additional ACE2 deficiency after viral invasion might amplify the dysregulation between the 'adverse' ACE→Angiotensin II→AT1 receptor axis and the 'protective' ACE2→Angiotensin1-7→Mas receptor axis. In the lungs, such dysregulation would favor the progression of inflammatory and thrombotic processes triggered by local angiotensin II hyperactivity unopposed by angiotensin1-7 (Hoffmann et al., 2020). ACE 2 is important in the fact that it is where SARS-CoV-2 binds. ACE2 amino acids form a grooved like pocket that the spikes of SARS-CoV-2 fit into or "bind." This is where SARS-CoV-2 hijacks the cell and begins to replicate creating the infection that creates COVID-19. Therefore, the more ACE2 expression, the more binding sites the subject coronavirus has to invade and spread. By modulating ACE2 expression in gateway tissues we reduce the coronavirus's ability to invade cells both decreasing disease susceptibility and also reducing entry points in currently infected individuals.

While ACE2 is the receptor for viral entry, TMPRSS2 primes viral spike proteins, and is therefore crucial for SARS-CoV2 entry into host cells. Recent studies revealed that TMPRSS inhibitors blocked virus entry. Several C. sativa extracts have shown to down-regulate TMPRSS2 gene expression in EpiOral and EpiIntestinal tissues (Wang et al., 2020).

The disclosed components also have been shown to stimulate endogenous Interleukein-1 receptors to release pro anti-inflammatory cytokine Il-1Ra, enhancing the release of the anti-inflammatory cytokines IL-4, IL-10, and IL-6 myokine. This stops the "Cytokine Storm" COVID-19 creates.

The disclosed compounds may be utilized to reduce symptoms of COVID-19 by modulating the immune system in multiple ways. At least one of the disclosed compounds comprises natural, GRAS (generally regarded as safe) by the FDA, sold as nutritional supplements, or are endogenous neurochemicals. The disclosed compounds may focus on the endocannabinoid system. cannabinoids have demonstrated the ability to downregulate cytokine and chemokine production and upregulate T-regulatory cells (Tregs) to suppress inflammatory responses. The endocannabinoid system is also involved in immunoregulation. For example, administration of endocannabinoids or use of inhibitors of enzymes that break down the endocannabinoids led to immunosuppression and recovery from immune-mediated injury to organs such as the liver. Manipulation of endocannabinoids and/or use of exogenous cannabinoids *in vivo* can constitute a potent treatment modality against inflammatory disorders.

The compounds disclosed herein may be formulated to target the human cannabinoid system. For example, the compound may target the classical cannabinoid receptors type 1 (CB₁) and 2 (CB₂), GPR55, and GPR119. Additionally, the disclosed compounds may target the IL-1B, as well as the TRPV ion channels. Examples of different types of eCBEs include fatty acid amide hydrolase (FAAH) inhibitors. This enhances the effects and duration cannabinoids in the human by body. The cannabinoids chosen as well as the eCBEs in the formulation may be naturally occurring. The disclosed compounds may utilize cannabinoids that have been shown to have a great affinity to the CB₂ receptor. Stimulation of CB₂ has been shown to reduce the inflammatory response in SARS-CoV-2 patients improve the overall condition of the patient. The stimulation of CB₂ controls the inflammatory cascade in several checkpoints, considering its capability to reduce the production of a large number of cytokines. Furthermore, by utilizing multiple cannabinoids from multiple sources and enhancing their effects with eCBEs, we create something commonly known as "the Entourage effect." The Entourage Effect is a proposed mechanism by which cannabis compounds, other than tetrahydrocannabinol (THC), act synergistically with it (each other) to modulate the overall effects of the plant (cannabinoids). In addition to the cannabinoid system, we target γ-Aminobutyric acid type A receptors (GABAARs) to reduce hypertension and give a sense of well being.

Some of the components of the disclosed compounds may be endocannabinoid enhancers (eCBE), which may be used to enhance the activity of the endocannabinoid system by increasing extracellular concentrations of endocannabinoids. The disclosed compounds may incorporate eCBEs and cannabinoids with a high affinity to the CB₂ receptor because stimulation of the CB₂ receptor can reduce the inflammatory response in people with SARS-CoV-2 and release interferons (IFNs). Targeting Cannabinoid receptors with agonists creates the IL-1ra receptor, which is an important anti-inflammatory cytokine. By targeting the cannabinoid system, the disclosed compounds may enhance the release of IL-1ra, which stimulates the immune system to produce anti-inflammatory cytokines. The production of the anti-inflammatory cytokines stops the release and production of new viruses. The disclosed compounds may also stimulate the release of "messengers" interferons, which tells the body what cell is infected as well as informing adjacent cells to increase their defenses. The disclosed compounds may be formulated to target the gamma-aminobutyric acid type A receptors (GABA_{A}R), which reduces hypertension and provides a sense of well-being.

Proinflammatory cytokines play a central role in inflammatory diseases of infectious or noninfectious origin. PAMPs and DAMPs trigger a cytokine cascade that initially is composed of the proinflammatory cytokines (IL-1, IL-6, IL-8, IL-12, IFN-γ, IL-18, and TNF itself) (Srinivasan et al., 2017). These cytokines serve to contain and resolve the inflammatory foci through activation of local and systemic inflammatory responses. TNF also triggers a cytokine cascade of the antiinflammatory cytokines that block proinflammatory cytokine synthesis, as well as cytokine inhibitors that block proinflammatory cytokine actions. In most cases the inflammatory response is successfully resolved. Overzealous production of cytokines or the inability to shut down proinflammatory cytokine production, however, can lead to increasing concentrations of cytokines in the systemic circulation ("cytokine storm"). This continued cytokine production can have a deleterious effect on the host, with the development of hypotension, intravascular thrombosis, pulmonary edema, and hemorrhage; if this process is left unchecked, it can lead to multiple organ failure and death. This condition often is referred to as the systemic inflammatory response syndrome (SIRS). This term describes the clinical manifestations of widespread endothelial inflammation that leads to increased vascular permeability. This condition is the initiating pathologic process in a group of diverse disorders, such as bacterial sepsis, ischemia, burn injury, trauma and tissue injury, and hemorrhagic shock.

It has become apparent that interactions between pro-inflammatory and anti-inflammatory mediators regulate the inflammatory response. Anti-inflammatory cytokines, in particular IL-10, inhibit proinflammatory cytokine synthesis and adhesion molecule expression while increasing the levels of specific cytokine inhibitors. Excess production of anti-inflammatory cytokines, however, can compromise the host's ability to clear microorganisms through suppression of immune cell function. If a balance is not maintained, the result is either an excessive proinflammatory response or immunosuppression and increased susceptibility to secondary infection. Thus, a cytokine cascade may be beneficial to the host by initiating the inflammatory response; however, overproduction or underproduction of proinflammatory or anti-inflammatory endogenous mediators may actually be deleterious to the host (Srinivasan et al., 2017).

To date, therapeutic strategies targeting proinflammatory cytokines such as TNF and IL-1β have proved ineffective in the treatment of SIRS-multiple clinical trials of antagonists of proinflammatory mediators demonstrated no improvement, and in some cases, worsened survival. Proinflammatory cytokines are critical to the initiation of the inflammatory response; however, their levels may have peaked before the clinical signs and symptoms of SIRS become apparent. Furthermore, although a hyperinflammatory response may be responsible for some of cases of sepsis-related death, a predominant anti-inflammatory response or global cytokine suppression may be the cause in many other instances, especially in populations with weakened immune systems such as neonates or the elderly. As a result, recent therapeutic approaches have focused more on immunomodulatory or immune-stimulatory mediators, such as granulocyte-monocyte colony-stimulating factor or IL-7, which has an important role in lymphocyte replenishment. Furthermore, mediators that appear later in disease progression may also hold promise for therapeutic intervention in uncontrolled inflammation in the context of severe sepsis and autoimmune disorders. DAMPs such as HMGB1, mitochondrial DNA and heat shock proteins, and mitochondrial formyl peptide are important late proinflammatory mediators. HMGB1, originally identified as a DNA-binding protein, is now recognized as a late mediator of sepsis and SIRS. HMGB1 is actively released by macrophages and endothelial cells during the inflammatory response, as well as passively from necrotic cells. HMGB1 mediates numerous proinflammatory actions both locally and systemically. Antibodies or antagonists directed against HMGB1 are protective in animal models of sepsis and SIRS.

Another late proinflammatory mediator, macrophage inhibitory factor was originally identified as a modulator of macrophage migration; it is now recognized to be a critical regulator of the inflammatory response. In animal models of infection and sepsis, anti-macrophage inhibitory factor therapy significantly improved survival. Thus, these "late" proinflammatory mediators may provide novel therapeutic targets for the treatment of SIRS. Strategies that selectively target DAMP-related inflammatory responses, while allowing appropriate immune response to PAMPs, are of especial interest in the context of SIRS and sepsis. Both exogenous and endogenous cannabinoids inhibit proinflammatory cytokine production by macrophages stimulated through Toll-like Receptors (TLRs). TLRs play a crucial role in macrophages sensing danger to trigger inflammatory responses. Still further, Manuka honey's antibacterial properties, which are what set it apart from traditional honey, may also be introduced in an exemplary discloed formulation. Methylglyoxal is its active ingredient and likely responsible for these antibacterial effects. Additionally, manuka honey has antiviral, anti-inflammatory and antioxidant benefits

### Formulation of the Composition

In a general embodiment, the composition is an emulsion formed from active ingredients mixed with a pharmaceutically acceptable carrier. The active ingredients can include hemp extract, a cannabinoid enhancer, a fatty acid amide, a kava extract, and an alkaloid. In a more particular embodiment, the pharmaceutically acceptable carrier is a medium-chain triglyceride (MCT), the cannabinoid enhancer is oleamide, the fatty acid amide is palmitoylethanolamide (PEA), and the alkaloid is piperine. In some embodiments, the composition can include lecithin.

The MCT may be caproic acid, caprylic acid, capric acid, lauric acid, or any combination thereof. MCTs for the compounds disclosed herein may be obtained from natural sources such as coconut oil and/or palm kernel oil through various separation techniques known in the art. The MCT may be used as a solvent for the preparation of the compounds disclosed herein. For example, the compounds disclosed herein may be prepared in tincture format in an MCT. It may be advantageous to use the tincture medium to facilitate sublingual mucosal absorption, which aids in situations when a patient is intubated.

The kava extract may comprise one or more kavalactones. The kavalactone in the kava extract may be desmethoxyyangonin, methysticin, yangonin, dihydromethysticin, dihydrokavain, kavain, 10-methoxyyangonin, 11-methoxyyangonin, 11-hydroxyyangonin, 11-methoxy-12-hydroxydehydrokavain, 7,8-dihydroyangonin, 5-hydroxykavain, 5,6-dihydroyangonin, 7,8-dihydrokavain, 5,6,7,8-tetrahydroyangonin, 5,6-dehydromethysticin, 7,8-dihydromethysticin, or any combination thereof.

Kavalactones have demonstrated an effectiveness in alleviating anxiety. For example, kavain has demonstrated an ability to positively modulate all receptors regardless of the subunit composition. Kavain has demonstrated a greater degree of enhancement at α4β2δ GABAARs. Kavalactones have also demonstrated an ability to induce attenuation of the a- and g-spinal motor systems directed by supraspinal sites and are also reported to be an inhibitor of CYP450 enzymes (CYP1A2, 2C9, 2C19, 2D6, 3A4 and 4A9/11), which facilitates muscle relaxation. In addition, kavalactones can inhibit calcium channels, and various kavalactones may do so additively, producing a reduction of calcium influx by as much as 70 percent. Thus, kavalactones may facilitate broad inhibition of neuronal firing.

Several kavalactones have also been found to inhibit sodium channels, further contributing to the inhibitory effect. Kavalactones may have other beneficial psychoactive properties. Kavalactones have also demonstrated an ability to reversibly block platelet MAO B enzymes. Kavain has demonstrated an ability to be a good potency *in vitro* inhibitor of human MAO-B. Kavain interacts reversibly and competitively with MAO-A and MAO-B. Yangonin has demonstrated an ability to be a particularly potent MAO inhibitor for MAO-A and MAO-B. Thus, some of the central effects (e.g., anxiolytic) of kavalactones may be mediated by MAO inhibition. Kava-kava extract has the ability to be a reversible inhibitor of MAO-B in intact platelets and disrupted platelet homogenates. Structural differences of kava pyrones result in different potency of MAO-B inhibition. In at least one example, the order of potency of select kava pyrones was desmethoxyyangonin >methysticin>yangonin>dihydromethysticin> dihydrokavain > kavain. In this example, the two most potent kava pyrones (desmethoxyyangonin and methysticin) facilitated a particularly high inhibition pattern. Thus, it may be advantageous to include kava pyrone-enriched extracts for the inhibition of MAO-B for their psychotropic activity.

The functional properties of a major anxiolytic kavalactone, kavain at human recombinant, may include α1β2, β2γ2L, αxβ2γ2L (x = 1, 2, 3 and 5), α1βxγ2L (x = 1, 2 and 3) and α4β2δ GABAARs expressed in Xenopus oocytes using the two-electrode voltage clamp technique. Kavain has demonstrated an ability to positively modulate all receptors regardless of the subunit composition but has shown a higher degree of enhancement at α4β2δ than at α1β2γ2L GABAARs (Ligresti et al., 2012). Yangonin has exhibited an affinity for the human recombinant CB₁ receptor with a *K*ᵢ = 0.72 µM and selectivity vs. the CB₂ receptor (*K*ᵢ > 10 µM). The CB₁ receptor affinity of yangonin indicates that the endocannabinoid system might contribute to the complex human psychopharmacology of the traditional kava drink and the anxiolytic preparations obtained from the kava plant.

Lecithin is known in the art to comprise glycolipids, triglycerides, and phospholipids. Examples of suitable phospholipids may be phosphate-dylcholine, phosphatidylethanolamine, and phosphatidyli-nositol. Soybean lecithin has demonstrated an ability to effect encapsulation, controlled release, and successful delivery of the curative factors to intracellular regions in which they procure these properties from their flexible physicochemical and biophysical properties, such as large aqueous center and biocompatible lipid, self-assembly, tunable properties, and high loading capacity. SARS-CoV2 uses the lungs as its powerplant to replicate. Soy lecithin may be used to increase the half-life and delivery, for an aerosol or possibly vaporizable product, to target the lungs.

Soy lecithin liposomes, as drug carriers, have demonstrated an ability to treat tuberculosis (TB). Soy lecithin liposomes can provide the additional biological mechanism of achieving targeted administration of anti-TB drugs at lower dosages and with minimal side effects while circumventing the drug resistance mechanisms of *M. tuberculosis* strains. Nanodevices, such as liposomes, provide the much-needed biological mechanism of achieving targeted administration of anti-TB drugs at lower dosages and with minimal side effects while circumventing the drug resistance mechanisms of M. tuberculosis strains. In some instances, inhaled drugs may be preferable as therapeutic strategies because they are able to reach the cavitary lesions of the bronchial tree where bacteria are overtly present and where strains of *M. tuberculosis* rapidly multiply. With the help of liposomes, the half-life and targeting efficiency of anti-TB therapies can be enhanced when compared to inhalable dry powder formulations with no liposomes. However, previous studies have recorded certain difficulties in anti-TB drug entrapment in liposomes. In one example, when ethionamide was used for incorporation in the lipid film, the trapping efficiency increased to 42%, but the equivalent molar ratio of drug: lipid of 0.04 was too low to achieve the expected therapeutic benefits. Liposomes are artificial vesicles of smaller spherical shape that can be produced from natural phospholipids and non-toxic cholesterol (Cruz et al., 2009) were developed to improve the biodistribution of compounds at specific locations in the body. Thus, they became recognized as carriers of biologically active compounds, with the ability to enhance and/or modify the activity of the compounds with which they are associated. This effect depends on the chemical composition and the phospholipid structure (Machado et al., 2014). One method of preparation of liposomes DRVs type, based on the dehydration and rehydration process comprises mixing a suspension of small empty liposomes (prepared in water), freeze-dried after mixing. The preparation of this rehydration under specific conditions of temperature (>Tt) and lipid concentration leads to obtaining liposomes with a high encapsulation rate, referred to as DRVs ("dehydration rehydration vesicles") and allow a high rate of encapsulation (Frézard et al., 2005). The classic method of lipid film hydration for production of nanosized liposomes remains used due to the simplicity and low cost (Mertins, 2004). In the present disclosure, atomization, lyophilization, agitation, sonication and freeze-thaw extrusion were applied to standardize the structures, as complementary techniques.

The hemp extract may comprise one or more cannabinoids. Cannabinoids act on glia and neurons to inhibit the release of pro-inflammatory molecules, including interleukin-1 (IL-1), tumor necrosis factor (TNF) α, and nitric oxide (NO) (Molina-Holgado et al., 1997, 2002; Shohami et al., 1997; Puffenbarger et al., 2000; Cabral et al., 2001), and enhance the release of the anti-inflammatory cytokines IL-4, IL-10 (Klein et al., 2000), and IL-6 (Molina-Holgado et al., 1998). Specifically, targeting Cannabinoid receptors with agonists creates IL-1ra which is an important anti-inflammatory cytokine. It is notable however, both CB₁ and CB₂ receptors modulate release of endogenous IL-1ra. A neuroprotective mechanism of action for CBs may be used in response to inflammatory or excitotoxic insults that is mediated by both CB₁ and CB₂ receptor-dependent pathways. Furthermore, the anti-inflammatory cytokine IL-1ra is an essential mediator of CB actions on neurons and glia and that both CB₁ and CB₂ receptors modulate the release of IL-1ra from primary cultured glial cells. Therefore, by targeting the cannabinoid system we may mediate Il-1 by enhancing release of Il1-ra stimulating the immune system to produce anti-inflammatory cytokines mediating the actions of PLpro stopping the release and production of new viruses. The disclosed compounds and/or formulations may stimulate the release of the body's cell "messengers" Interferon, which tells the body what cell is infected as well as tells adjacent cells to step up their defenses

The cannabinoids in the hemp extract may be any one of the N-acylethanolamines, kaempherol, any of the N-alkylamides, rutamarin, 3,3'-Diindolylmethane, virodhamine, guineesine, cannabidiol (CBD), any of the tetrahydrocannabinol (THC) isomers, any one of the terpenes, humulene, or any combination thereof. Some examples of the functional groups bonded to N-acylethanolamine may include linoleoyl, oleoyl, and palmitoyl. N-acylethanolamines may act as FAAH inhibitors. N-acylethanolamines may also target GPR55 receptors. Kaempherol may act as MAGL and FAAH inhibitors in varying concentrations. For example, kaempherol may be therapeutically effective as a MAGL inhibitor in a concentration of IC₅₀ <100 nM. Additionally, kaempherol may be therapeutically effective as a FAAH inhibitor in a concentration of IC₅₀<1 µM. N-alkylamides exhibit a selective affinity for CB₂ receptors at varying concentrations. For example, N-alkylamides may be therapeutically effective at selecting the CB₂ receptors at a concentration of Kᵢ value <100 nM. N-alkylamides also exhibit the ability to target (ECS) PPARs, Ion channels, Inhibition of AEA transport, Partial FAAH inhibitor. Rutamarin exhibits a selective affinity for CB₂ receptors at varying concentrations. For example, rutamarin may be therapeutically effective at selecting the CB2 receptors at a concentration of Ki value <10 µM. 3,3'-Diindolylmethane exhibits a selective affinity for CB₂ receptors at varying concentrations. For example, 3,3'-Diindolylmethane may be therapeutically effective at selecting the CB2 receptors at a concentration of Ki value ≅1 µM. 3,3'-Diindolylmethane is a partial agonist at CB2 receptor. Virodhamine (O-arachidonoyl ethanolamine; O-AEA) is an endocannabinoid and a nonclassic eicosanoid. An endocannabinoid enhancer (eCBE) is a type of cannabinoidergic drug that enhances the activity of the endocannabinoid system by increasing extracellular concentrations of endocannabinoids. Examples of different types of eCBEs include fatty acid amide hydrolase (FAAH) inhibitors. This enhances the effects and duration cannabinoids in the human by body.

O-Arachidonoyl ethanolamine is arachidonic acid and ethanolamine joined by an ester linkage, the opposite of the amide linkage found in anandamide. Virodhamine acts as an antagonist of the CB₁ receptor and agonist of the CB₂ receptor. Concentrations of virodhamine in the human hippocampus are similar to those of anandamide, but they may be 2 to 9-fold higher in peripheral tissues that express CB₂. O-AEA is a inhibitor of CYP2J2 epoxygenase. Together, the role of O-AEA as a eCB inhibitor of CYP2J2 may control of the activity of cardiovascular CYP2J2 *in vivo* and potentially cross talk between the cardiovascular endocannabinoids and cytochrome P450 system. Guineensine may act as a cannabinoid transport modulator. Guineensine may inhibit the cellular reuptake of anandamide and 2-arachidonoylglycerol. This can cause an increase in the activity of the two neurotransmitters which are classified as cannabinoids. Guineesine can dose-dependently produce cannabimimetic effects, which are indicated by potent catatonic, analgesic, hypo-locomotive and hypo-thermic effects . Guineesine is also a monoamine oxidase inhibitor (MAOI) in vitro at varying concentrations. For example, guineesine may be therapeutically effective at IC₅₀=139.2 µM. Guineensine has exhibited an ability to inhibit proinflammatory cytokine production in endotoxemia . Accordingly, it is advantageous to include guineesine in the disclosed compounds.

CBD has demonstrated an ability to modulate the inflammatory processes through a CB₂-dependent mechanism. CBD can induce CB₂ activation indirectly by increasing AEA levels. CBD exerts its anti-inflammatory properties by reducing pro-inflammatory cytokines. CBD has demonstrated an ability to act as an immune suppressive with mechanisms that may involve direct suppression of activation of various immune cell types, induction of apoptosis, and promotion of regulatory cells, which, in turn, control other immune cell targets. Targets of suppression may include cytokines such as TNF-α, IFN-γ, IL-6, IL-1β, IL-2, IL-17A, and chemokines, such as CCL-2. Generally, CBD can act to suppress target cells, such as effector T cells and microglial cells, through suppression of kinase cascades and various transcription factors. For example, CBD-induced suppression of phosphorylated p38 may lead to compromised AP-1 or NF-κB activity. Direct suppression of target cells may also include induction of IκB, which could contribute to decreased NF-κB activity. The involvement of regulatory cell induction by CBD is also a major part of the mechanism by which CBD controls immune responses, and CBD has been shown to induce Tregs and MDSCs. Finally, CBD-induced apoptosis is likely an important mechanism in many target cells. Additionally, Δ⁹-tetrahydrocannabinol (Δ⁹-THC) may act potently at TRPV2, moderately modulates TRPV3, TRPV4, TRPA1, and TRPM8, and Cb1. Values for THC: CB₁ Affinity (Ki)=10nM partial agonist; CB₂ Affinity (Ki)=24nM partial agonist. Table 1 illustrates receptors involved in mediating cannabidiol effects.

| Table 1 | |
|---|---|
| Receptor | Activity |
| CB₁ | Agonist |
| CB₂ | Agonist |
| FAAH | Inhibition |
| TRPV1 | Agonist |
| Adenosine A_{2A} | Agonist |
| PPAR-gamma | Activation |
| 5-HT1a | Agonist |
| GPR55 | Antagonist |

Some examples of terpenes that may be included in the composition may be beta-carophyllene ((E)-BCP) and/or alpha-humulene. (E)-BCP may selectively bind to the CB2 receptor (Kᵢ = 155 ± 4 nM), which can make (E)-BCP a functional CB2 agonist. Upon binding to the CB2 receptor, (E)-BCP may inhibit adenylate cylcase, which results in intracellular calcium transients and weakly activates the mitogen-activated kinases Erk1/2 and p38 in primary human monocytes. (E)-BCP may also inhibit lipopolysaccharide (LPS)-induced proinflammatory cytokine expression in peripheral blood and attenuates LPS-stimulated Erk1/2 and JNK1/2 phosphorylation in monocytes. (E)-BCP is a functional nonpsychoactive CB2 receptor ligand in foodstuff and a macrocyclic antiinflammatory cannabinoid. (E)-BCP has demonstrated an ability to be orally bioavailable. Thus, it would be advantageous to include (E)-BCP for oral consumption. Humulene, also known as α-caryophyllene or alpha-humulene, is a ring-opened isomer of β-caryophyllene. Humulene has demonstrated an ability to be an effective anti-inflammatory activity. Humulene possesses both topical and systemic anti-inflammatory properties (Chaves et al., 2008) and is an effective analgesic when taken topically, orally, or by aerosol (Rogerio et al., 2009). Humulene can result in an antineoplastic effect by inducing apoptosis. Beta-Caryophyllene can be used synergistically (Legault and Pichette, 2007). Humulene, also known as α-caryophyllene, is a ring-opened isomer of β-caryophyllene. Humulene possesses powerful anti-inflammatory activity equal to dexamethasone in an animal model (Fernandes et al., 2007). Humulene has demonstrated an ability to increase secretion of IL-8, a chemokine with various functions, including promoting angiogenesis, helpful in wound healing but not typically associated with anticancer compounds (Satsu et al., 2004).

Piperine has demonstrated chemopreventive and antioxidant activities. Additionally, piperine has also demonstrated immunomodulatory, anticarcinogenic, stimulatory, hepatoprotective, antiinflammatory (Darshan and Doreswamy 2004), antimicrobial (Yang et al 2002), and antiulcer activities (Bai and Xu 2000). Piperine also has biotransformative effects and can enhance the bioavailability of different drugs such as rifampicin, sulfadiazine, tetracyline, and phenytoin by increasing their absorption, by slowing down the metabolism of the drug, or by a combination of the 2 (Atal and others 1985; Wu 2007). Piperin may stimulate the digestive enzymes of the pancreas, protect against oxidative damage, lower lipid peroxidation, and enhance the bioavailability of a number of therapeutic drugs. Further, the anti-inflammatory activities of piperine have been demonstrated in rat models of carrageenan-induced rat paw edema, cotton pellet-induced granuloma, and a croton oil-induced granuloma pouch. Constituents of the piper species have shown *in vitro* inhibitory activity against the enzymes responsible for leukotriene and prostaglandin biosynthesis, 5-lipoxygenase and COX-1, respectively. Thus, it is advantageous to incorporate piperin to treat inflammatory diseases that are accompanied by severe pain. A component of pungency by piperine results from activation of the heat- and acidity-sensing TRPV ion channels, TRPV1 and TRPA1, on nociceptors, the pain-sensing nerve cells. Piperine has demonstrated the ability to inhibit the expression of IL6 and MMP13 and reduce the production of PGE₂ in a dose dependent manner at varying concentrations. For example, piperine may be therapeutically effective at concentrations between about 10 and about 100 µg/ml. In another example, piperine has been therapeutically effective at inhibiting PGE₂ at a concentration of about 10 µg/ml of piperine. Thus, piperine has a demonstrated ability to produce anti-inflammatory, antinociceptive, and antiarthritic effects via Il-1b (member of IL family of cytokines). Further, Piperine can increase bioavailability of various drugs ranging from 30% to 200%. Thus, it is advantageous to incorporate piperine in a therapeutic compound to help regulate immune function with the intention of stopping negative effects of ailments such as SARS CoV-2.

Piperine can also activate the TPRV ion channels. These channels modulate ion entry, mediating a variety of neural signaling processes implicated in the sensation of temperature, pressure, and pH, as well as smell, taste, vision, and pain perception. Many diseases involve TRP channel dysfunction, including neuropathic pain, inflammation, and respiratory disorders. Cannabinoids have demonstrated an ability to modulate a certain subset of TRP channels. The TRP vanilloid (TRPV), TRP ankyrin (TRPA), and TRP melastatin (TRPM) subfamilies were all found to contain channels that can be modulated by several endogenous, phytogenic, and synthetic cannabinoids. At least six TRP channels from the three subfamilies mentioned above have been reported to mediate cannabinoid activity: TRPV1, TRPV2, TRPV3, TRPV4, TRPA1, and TRPM8. Piperine is slightly soluble in water (40 mg/L at 18 °C; Vasavirama and Upender 2014). The low solubility of piperine in water and its poor dissolution is rate-controlling in the absorption process of piperine. Pharmaceutical activities of piperine may be limited due to its low water solubility and because use of it at high concentrations can be toxic for the central nervous and reproductive systems (Veerareddy and others 2004; Pachauri and others 2015). In some examples of the compounds disclosed herein, a lipid encapsulation of piperine may be incorporated to increase the bioavailability piperine and the other components of the compound.

Cis-9,10-octadecanoamide (oleamide, ODA) may be used as a sleep-inducing substance (Cravatt et al., 1995). An 'entourage' effect was suggested (Lambert & Di Marzo, 1999). ODA may potentiate or prolong the effects of endocannabinoids such as AEA by competitively inhibiting the enzyme FAAH (Mechoulam et al., 1997). Furthermore, ODA may act as a full cannabinoid CB₁ receptor agonist. Therefore, in addition to allosteric modulation of other receptors and possible entourage effects due to fatty acid amide hydrolase inhibition, the effects of ODA may be mediated directly *via* the CB₁ receptor. Some investigations of ODA have revealed decreased protein levels and metabolic activities of CYP1A2, CYP2B, and CYP2C11, along with a drop in metabolic activity of CYP2D2. Oleamide has not exhibited a tendency to interact with human pregnane X, constitutive androstane, or aryl hydrocarbon receptors in reporter gene experiments and did not regulate their target P450 genes in primary human hepatocytes. In vitro oleamide is neither an agonist nor antagonist of major human nuclear receptors involved in the regulation of xenobiotic metabolism.

Palmitoylethanolamide (PEA) is a fatty acid amide, belonging to the class of nuclear factor agonists. PEA has demonstrated an ability to bind to a nuclear receptor through which it exerts a variety of biological effects, some related to chronic inflammation and pain. In some circumstances, PEA has exhibited a tendency to target the peroxisome proliferator-activated receptor alpha (PPAR-α). PEA also has exhibited an affinity to cannabinoid-like G-coupled receptors GPR55 and GPR119. Generally, PEA may not exhibit an affinity for the cannabinoid receptors CB₁ and CB₂. However, the presence of PEA (or other structurally related N-acylethanolamines) tends to enhance anandamide activity through the "entourage effect." Further, PEA may directly or indirectly stimulate CB₂ receptors (Re, Barbero, Miolo, & Di Marzo, 2007). PEA has also exhibited the ability to bind to CB₁ receptors (Lin, Lu, Wu, Huang, & Wang, 2015). PEA and OEA tend to exert their effects through the proliferator-activated receptor alpha (PPARα) or GPR119 (Hansen & Artmann, 2008). PEA has also demonstrated the ability to improve all macroscopic signs of colitis and reduce proinflammatory cytokines. In situations where there is acute or chronic inflammation, PEA levels are altered, and the endocannabinoid system (ECS) tends to be imbalanced. In at least one instance, the deregulation of cannabinoid receptors and their endogenous ligands accompanies the development and progression of β-amyloid-induced neuroinflammation. PEA has also demonstrated the ability to have anti-inflammatory, antinociceptive, neuroprotective, and anticonvulsant properties.

Anandamide (AEA), PEA, and oleoylethanolamide (OEA) are synthesized from the membrane's phospholipids by N-acylphosphatidylethanolamine-specific phospholipase D (NAPE-PLD). PEA and OEA do not bind to CB₁R, but they can enhance AEA activity at transient receptor potential channels of vanilloid type-1 (TRPV1). AEA, PEA, and OEA are all degraded by fatty acid amide hydrolase (FAAH). OEA and PEA can increase AEA levels by competing with AEA for FAAH (mainly OEA) or by downregulating FAAH expression (mainly PEA). Cannabidiol (CBD), a non-psychoactive component of the cannabis plant, activates peroxisome proliferator-activated receptors (PPARs) and TPRV1 and inhibits FAAH and hence might compensate for lower levels of AEA, OEA, and PEA in children with ASD.

The disclosed compounds may include Epigallocatechin gallate (EGCG), which is also known as epigallocatechin-3-gallate. EGCG has demonstrated an affinity for the CB₁ receptor. EGCG is a CB₂ agonisht and a modulator of the GABAA receptor 44. The disclosed compounds may also include Biochanin A. Biochanin A is generally known as a flavonoid. Biochanin A is a FAAH inhibitor. Biochanin A has not demonstrated a tendency to interact to any major extent with CB₁ or CB₂ receptors, nor with FAAH-2. Biochanin A has demonstrated an ability to inhibit the hydrolysis of 0.5 µM AEA FAAH with IC₅₀ values ranging from about 1.8 to about 2.4 µM. Biochanin A has demonstrated an ability to inhibit the spinal phosphorylation of extracellular signal-regulated kinase produced by the intraplantar injection of formalin. The effects of both compounds were significantly reduced by the CB₁ receptor antagonist/inverse agonist AM251 (30 µg i.pl.). Biochanin A (15 mg·kg⁻¹ i.v.) has not demonstrated an ability to increase brain AEA concentrations but has produced a modest potentiation of the effects of 10 mg·kg⁻¹ i.v. AEA.

The disclosed compounds include at least one of the following flavonoids: taxifolin, morin, quercetin, fisetin, apigenin, and galangin. The disclosed flavonoids have demonstrated the ability to inhibit enzymes correlated to viral infections and autoimmune diseases. For example, the disclosed flavonoids have exhibited the ability to inhibit the MAOB enzyme, which exhibits elevated levels during an illness (e.g., coronavirus infection, autoimmune diseases, and cancer). Apigenin is a common dietary flavonoid that is abundantly present in many fruits, vegetables and Chinese medicinal herbs and serves multiple physiological functions, such as strong anti-inflammatory, antioxidant, antibacterial and antiviral activities and blood pressure reduction. Apigenin has demonstrated an ability to suppress various human cancers in vitro and in vivo by multiple biological effects, such as triggering cell apoptosis and autophagy, inducing cell cycle arrest, suppressing cell migration and invasion, and stimulating an immune response. Apigenin has demonstrated an ability to be developed either as a dietary supplement or as an adjuvant chemotherapeutic agent for cancer therapy. And as introduced above, Manuka honey's antibacterial properties, and antiviral, anti-inflammatory and antioxidant benefits, may be added to formulations targeting cancer.

The disclosed compounds may include curcumin. Curcumin (and resveratrol) have demonstrated an ability to suppress constitutive activation of STAT3, through upregulation of PIAS3. Curcumin may function as a MAO inhibitor (MAO-A and MAO-B).

The composition for treating an infection of SARS-CoV-2 by targeting cannabinoid receptors may comprise an emulsion formed from a pharmaceutically acceptable carrier mixed with active ingredients. The pharmaceutically acceptable carrier may be between 15-85 wt% of the composition. In an embodiment where the composition is in liquid form, the pharmaceutically acceptable carrier may be between 50-85 wt%. In another embodiment where the composition is in gel form, the pharmaceutically acceptable carrier may be between 15-35 wt%. The active ingredients may comprise: an effective amount of a hemp extract to provide a source of endocannabinoids; an effective amount of an endocannabinoid enhancer to inhibit endocannabinoid hydrolases; an effective amount of a fatty acid amide to enhance endocannabinoid activity via an entourage effect; an effective amount of a kava extract to alleviate anxiety; and an effective amount of an alkaloid to enhance bioavailability of one or more of the active ingredients.

The pharmaceutically acceptable solvent may be a medium-chain triglyceride. The medium-chain triglyceride may be derived from oils such as palm kernel oil and coconut oil. For example, extracts from oils may be hexanoic acid, octanoic acid, decanoic acid, dodecanoic acid, or any combination thereof. In one example, the cannabinoid enhancer may be oleamide. The fatty acid may be at least one of PEA and virodhamine. The alkaloid may be piperine.

The effective amount of the hemp extract may be between 5-40 wt% of the composition. In an embodiment where the composition is in liquid form, the hemp extract may be between 5-13 wt%. In another embodiment where the composition is in gel form, the hemp extract may be between 15-40 wt%. The effective amount of the fatty acid primary amide may be between 1.5-6 wt% of the composition. In an embodiment where the composition is in liquid form, the fatty acid primary amide may be between 1.5-4 wt%. In another embodiment where the composition is in gel form, the fatty acid primary amide may be between 2-6 wt%. The effective amount of the fatty acid amide may be between 1.5-11 wt% of the composition. In an embodiment where the composition is in liquid form, the fatty acid amide may be between 1.5-4 wt%. In another embodiment where the composition is in gel form, the fatty acid amide may be between 5-11 wt%. The effective amount of the alkaloid may be between 0.2-3 wt% of the composition. In an embodiment where the composition is in liquid form, the alkaloid may be between 0.2-3 wt%. In another embodiment where the composition is in gel form, the alkaloid may be between 0.5-3 wt%.

The kava extract may comprise at least one of desmethoxyyangonin, methysticin, yangonin, dihydromethysticin, dihydrokavain, kavain, 10-methoxyyangonin, 11-methoxyyangonin, 11-hydroxyyangonin, 11-methoxy-12-hydroxydehydrokavain, 7,8-dihydroyangonin, 5-hydroxykavain, 5,6-dihydroyangonin, 7,8-dihydrokavain, 5,6,7,8-tetrahydroyangonin, 5,6-dehydromethysticin, and 7,8-dihydromethysticin. The effective amount of the kava extract may be between 6.0-35 wt%. In an embodiment where the composition is in liquid form, the kava extract may be between 6.0-12.0 wt%. In another embodiment where the composition is in gel form, the kava extract may be between 15-35.0 wt%.

In some examples some of the active ingredients are at least partially encapsulated with lecithin, and the lecithin may be present in an amount from about 2 wt% of the composition. In an embodiment where the composition is in liquid form, the lecithin may be between 0.2-3 wt%. In another embodiment where the composition is in gel form, the lecithin may be between 0.5-3 wt%. The active ingredients that are at least partially encapsulated with lecithin may include the alkaloid.

The hemp extract may comprise at least one of cannabidiol (CBD), tetrahydrocannabinol (THC), cannabigerol, cannabinol, and terpenes. In examples where CBD is present, the CBD may comprise 99.5% of cannabinoids in the hemp extract. The hemp extract may comprise a full-spectrum CBD or a CBD isolate. An effective amount of the hemp extract may be 8 wt%. The hemp extract may comprise beta-caryphyllene in an amount between 0.005-0.03 wt% of the composition.

The disclosed compositions may include at least one of taxifolin, morin, quercetin, fisetin, apigenin, and galangin. In an embodiment, the disclosed compositions may include taxifolin in an amount of 0.1-3 wt%, morin in an amount of 0.6-4 wt%, quercetin in an amount of 1.0-6 wt%, fisetin in an amount of 2-8wt%, apigenin in an amount of 0.3-2 wt%, and galangin in an amount of 8-20 wt%. In another embodiment, the disclosed compositions may include curcumin in an amount of 1-15 wt%.

The effective amount of the ingredients in the disclosed compositions may vary depending on the form of the composition. Table 2 illustrates one example of an effective amounts for ingredients in the composition in liquid form. Table 3 illustrates one example of an effective amounts for ingredients in the composition in gel form.

| Table 2 | |
|---|---|
| Ingredients of Composition (Liquid) | Mass Percent of Composition |
| MCT | 50.0-85.0 |
| Lecithin | 0.2-3.0 |
| Oleamide | 1.5-4.0 |
| PEA | 1.5-4.0 |
| Piperine | 0.2-3.0 |
| CBD | 8.0-13.0 |
| Kavalactones | 6.0-12.0 |

| Table 3 | |
|---|---|
| Ingredients of Composition (Gel) | Mass Percent of Composition |
| MCT | 15.0-35.0 |
| Lecithin | 0.5-3.0 |
| Oleamide | 2.0-6.0 |
| PEA | 5.0-11.0 |
| Piperine | 0.5-3.0 |
| CBD isolate | 15.0-35.0 |
| Kavalactones | 15.0-35.0 |
| Beta-Caryophyllene | 0.005-0.03 |

### Manufacture of the Compositions

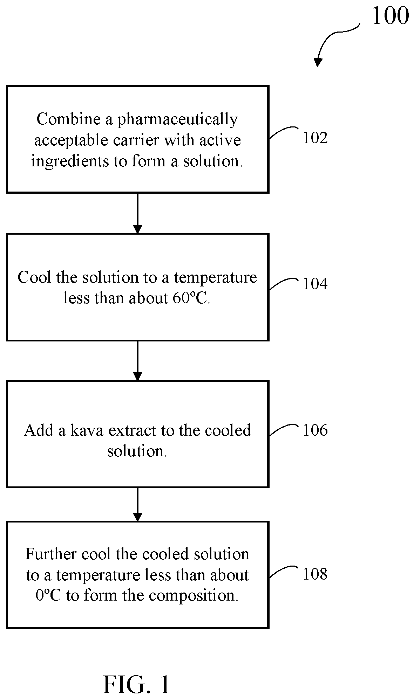

Referring to **FIGURE 1****,** a method is provided for manufacturing a composition according to an illustrative embodiment. Flowchart 100 begins at Step 102 where a pharmaceutically acceptable carrier is combined with active ingredients to form a solution. The active ingredients may include an effective amount of a hemp extract to provide a source of endocannabinoids, an effective amount of an endocannabinoid enhancer to inhibit endocannabinoid hydrolases, an effective amount of a fatty acid amide to enhance endocannabinoid activity via an entourage effect, an effective amount of a kava extract to alleviate anxiety, and an effective amount of an alkaloid to enhance bioavailability of one or more of the active ingredients. As used herein, the term "effective amount" means a sufficient amount of a compound that can significantly induce a positive modification the condition being treated, but low enough to avoid unwanted side effects, within the scope of sound judgment of a skilled artisan. The effective amount of a compound may vary with the particular condition being treated, the age and condition of the biological subject being treated, the severity of the condition, the duration of the treatment, and other factors within the knowledge and expertise of the skilled artisan.

In Step 104, the solution is cooled to a temperature less than about 60°C.

In Step 106, a kava extract is added to the cooled solution. In embodiments where the hemp extract comprises a cannabidiol isolate, a beta-caryphyllene may be added to the cooled solution along with the kava extract. In some embodiments, the cooled solution is emulsified to sufficiently disperse one or more of the active ingredients throughout the carrier. In one particular embodiment, emulsification occurs for about 1 minute.

In Step 108, the cooled solution is further cooled to a temperature less than about 0°C, thereby forming the composition. In one or more non-limiting embodiments, the solution is further cooled to a temperature of less than about 0°C steadily over a period of time between 5-10 hours. In other embodiments, the solution is further cooled to a temperature of less than about 0°C and then held for a period of time between 5-10 hours.

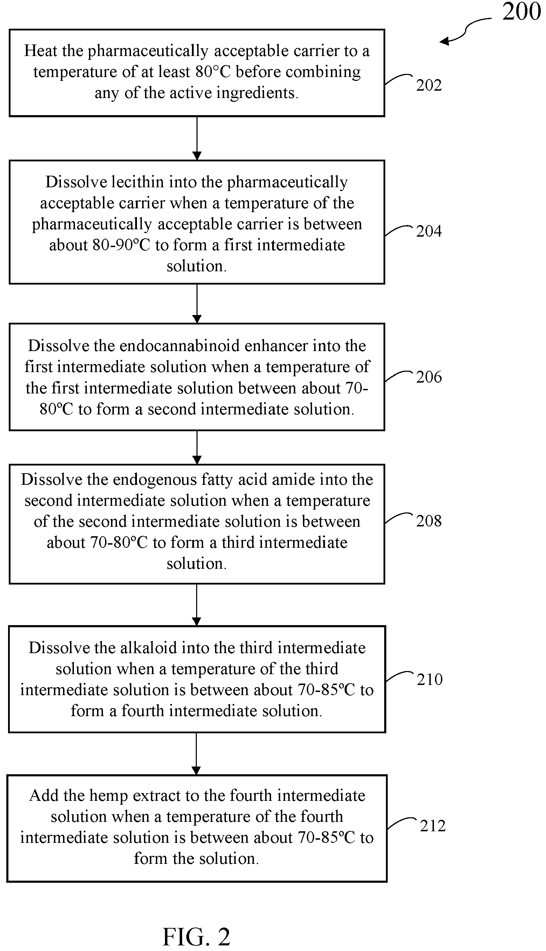

**FIGURE 2** is a flowchart of steps for combining a pharmaceutically acceptable carrier with active ingredients to form a solution according to an illustrative embodiment. Flowchart 200 begins at Step 202 where the solvent is heated to a temperature of about 80°C before combining any of the active ingredients with the pharmaceutically acceptable carrier.

In Step 204 the lecithin is dissolved into the pharmaceutically acceptable carrier when a temperature of the pharmaceutically acceptable carrier is between about 80°C and about 90°C to form a first intermediate solution.

In Step 206 the endocannabinoid enhancer is dissolved into the first intermediate solution when a temperature of the first intermediate solution between about 70°C and about 80°C to form a second intermediate solution.

In Step 208 the fatty acid amide is dissolved into the second intermediate solution when a temperature of the second intermediate solution is between about 70°C and about 80°C to form a third intermediate solution.

In Step 210 the alkaloid is dissolved into the third intermediate solution when a temperature of the third intermediate solution is between about 70°C and about 85°C to form a fourth intermediate solution.

In Step 212 the hemp extract is added to the fourth intermediate solution when a temperature of the fourth intermediate solution is between about 70°C and about 85°C to form the solution. In some embodiments, the solution is emulsified to sufficiently disperse the active ingredients throughout the carrier. In one particular embodiment, emulsification occurs for about 1 minute.

The disclosed principles may utilize different methods to achieve a degree of encapsulation in examples utilizing a tincture, including agitation (homogenization) and freezing. In other preparations, the methods may utilize inline sonication.

As disclosed herein, the compounds and treatments have been used with favorable results for coronaviruses (e.g., COVID-19), autoimmune diseases, pulmonary fibrosis, cancer and multiple sclerosis. The disclosed principles may also have favorable results with many other diseases. Positive results have been observed for Herpes Zoster Viral outbreaks (Shingles): Reduction of symptoms (perceived pain) from the individual experiencing in approximately 10 min. The disclosed principles have been a particularly effective treatment for "Shingles" pain. Therefore, the disclosed principles are also offered to treat Herpes Zoster as well as COVID-19. The disclosed principles may also work with systemic inflammatory response syndrome (SIRS). This term describes the clinical manifestations of widespread endothelial inflammation that leads to increased vascular permeability. Since this condition (SIRS) is the initiating pathologic process in a group of diverse disorders, such as bacterial sepsis, ischemia, burn injury, trauma and tissue injury, the disclosed principles should also be considered a treatment for those. Furthermore, disclosed principles have been used by some who suffer from genetic "autoimmune disorder" whereby the person "constantly hurts" or is "in constant pain" or "body and bones ache." These people claim to "feel better," "live a better quality of life," "feel normal for the first time" after ingesting the disclosed compositions. Individuals exhibited a sense of feeling better or "normal" within 10-30 min.

One inception of this disclosed formulation has been shown to combat COVID-19 with an onset and reduction of some symptoms in as little as 15min and a total sense of well-being after a few hours. The SARS-CoV-2 virus must overcome various defense mechanisms of the human body, including its non-specific or innate immune defense. During this process, infected body cells release messenger substances known as type 1 interferon. These attract natural killer cells, which kill the infected cells.

### Methods of Use

The disclosed compounds may be used to target multiple receptor sites to achieve immunomodulation. The disclosed compounds may target cannabinoid receptors type 1 (CB1) and 2 (CB2), GPR55, GPR119, PPAR-a, IL-1B, as well as the TPRV ion channels, GABBA, TLRs and Ras/Raf/MAPK signal pathways. The disclosed compounds reduce inflammatory response and stimulate the production of type I interferons, which are key antiviral mediators. By doing this, the disclosed compounds address the "tricks" Sars-Cov2 plays on our immune system. The disclosed compounds may incorporate cannabinoids that have been shown to have anti-inflammatory effects, since Sars-Cov2 has been shown to be highly pro-inflammatory. Stimulation of CB2 reduces the inflammatory response and for SARS-CoV-2 patients, improving the overall condition of the patient. The stimulation of CB2 controls the inflammatory cascade in several checkpoints, considering its capability to reduce the production of a large number of cytokines33. The use of the disclosed compounds may also utilize TRPV1 or vanilloid receptor agonists as the TRPV1 channel is involved in the regulation of calcium signaling, crucial for many cellular processes including proliferation, apoptosis, secretion of cytokines or T cell activation. Furthermore, TRPV1 appears as a polymodal receptor that takes part in cell-environment crosstalk. Consequently, it can influence not only cell behavior but also cell fate.68 The disclosed compounds may include other components that activate PPAR-a receptors to inhibit Fatty Acid Amide Hydrolaze (FAAH) enhancing the levels and actions of the endocannabinoid, anandamide, as well as all exogenous cannabinoids we introduce; again with the aim of decreasing inflammation and stimulating the production of type I interferon. The disclosed compounds may create something commonly known as "The Entourage Effect." The Entourage Effect is a proposed mechanism by which cannabis compounds, act synergistically with it (each other) to modulate the overall effects of cannabinoids. Inflammation, anxiety, and hypertension are common symptoms of COVID-19. Therefore, in addition to the cannabinoid system, the disclosed compounds may also include a natural ingredient that targets γ-Aminobutyric acid type A receptors (GABAARs). GABBA-A Receptors have close relation to inflammation and hypertension. GABAergic ingredient characteristics include antihypertension, antianxiety, and anti-inflammation. GABA, the principal inhibitory neurotransmitter in the adult brain, has a parallel inhibitory role in the immune system. Immune cells synthesize GABA and have the machinery for GABA catabolism. Antigen-presenting cells (APCs) express functional GABA receptors and respond electrophysiologically to GABA. Thus, the immune system harbors all of the necessary constituents for GABA signaling, and GABA itself may function as a paracrine or autocrine factor. GABAergic agents act directly on APCs, decreasing MAPK signals and diminish subsequent adaptive inflammatory responses in some models of Multiple Sclerosis as well. GABA receptor transcripts are present in immune cells and GABA treatment decreases inflammatory cytokine production in peripheral macrophages. GABA and GABA type A receptor (GABA-A-R) agonists decrease cytotoxic immune responses and cutaneous delayed-type hypersensitivity reactions. Treatment with GABA decreased T cell autoimmunity and the development of inflammatory responses in the non-obese diabetic mouse model of type 1 diabetes. The site of action of GABA in the adaptive immune response, however, remains obscure.

The SARS-CoV-2 virus must overcome various defense mechanisms of the human body, including its non- specific or innate immune defense. During this process, infected body cells release messenger substances known as type 1 interferon. These attract natural killer cells, which kill the infected cells. One of the reasons the SARS-CoV-2 virus is so 'successful' - and thus dangerous - is that it can suppress this non-specific immune response. In addition, it lets the invaded human cells produce the viral protein PLpro (papain-like protease). PLpro has two functions: It plays a role in the maturation and release of new viral particles, and it suppresses the development of type 1 interferons.

The disclosed compositions may be used to treat an infection of SARS-CoV-2. The release of Type 1 interferons triggers the immune system to respond to the viral infection. For example, endogenous Interleukein-1 receptors are stimulated to release the pro anti-inflammatory cytokine Il-1Ra while simultaneously enhancing the release of the anti-inflammatory cytokines IL-4, IL-10, and IL-6. The suppression of papain-like protease protein production reduces the ability of the coronavirus enzymes to process viral poly proteins to generate functional replicase complex and enable viral spread. The ACE2 expression modulation reduces the ability for the coronavirus to invade a cell. Additionally, ACE2 expression modulation decreases disease susceptibility and reduces entry points in infected individuals. The deregulation of TMPRSS2 gene expression reduces the ability for the coronavirus to replicate in the lungs. Additionally, deregulation of TMPRSS2 gene expression initiates pathology in the body.

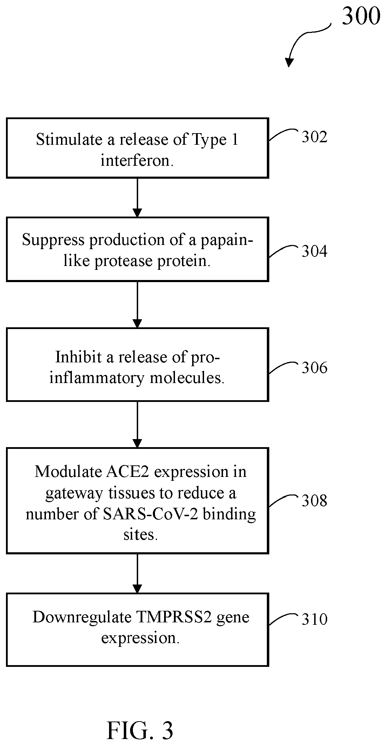

With reference now to **FIGURE 3****,** a method of treating an illness with an immunomodulating composition in accordance with an illustrative embodiment. Flowchart 300 begins at Step 302 by stimulating a release of Type 1 interferon. In Step 304, a production of papain-like protease protein is suppressed. In Step 306, a release of pro-inflammatory molecules is inhibited. In Step 308 ACE2 expression is modulated in gateway tissues to reduce a number of viral binding sites. In an illustrative embodiment, where the illness is an infection caused by SARS-CoV-2, ACE2 expression is modulated to reduce a number of SARS-CoV-2 binding sites. In Step 310 TMPRSS2 gene expression is downregulated.

While various embodiments in accordance with the principles disclosed herein have been described above, it should be understood that they have been presented by way of example only, and not limitation. Thus, the breadth and scope of this disclosure should not be limited by any of the above-described exemplary embodiments. Furthermore, the above advantages and features are provided in described embodiments, but shall not limit the application of such issued claims to processes and structures accomplishing any or all of the above advantages.

Additionally, the section headings herein are provided for organizational cues. These headings shall not limit or characterize the invention(s) set out in any claims that may issue from this disclosure. Specifically, and by way of example, although the headings refer to a "Technical Field," the claims should not be limited by the language chosen under this heading to describe the so-called field. Further, a description of a technology as background information is not to be construed as an admission that certain technology is prior art to any embodiment(s) in this disclosure. Neither is the "Summary" to be considered as a characterization of the embodiment(s) set forth in issued claims. Furthermore, any reference in this disclosure to "invention" in the singular should not be used to argue that there is only a single point of novelty in this disclosure.

Moreover, the Abstract is provided to allow the reader to quickly ascertain the nature of the technical disclosure. It is submitted with the understanding that it will not be used to interpret or limit the scope or meaning of the claims. In addition, in the foregoing Detailed Description, it can be seen that various features are grouped together in a single embodiment for the purpose of streamlining the disclosure. This method of disclosure is not to be interpreted as reflecting an intention that the claimed embodiments require more features than are expressly recited in each claim. Rather, as the claims reflect, inventive subject matter lies in less than all features of a single disclosed embodiment. Thus, the following claims are hereby incorporated into the Detailed Description, with each claim standing on its own as a separate embodiment.

## Claims

1. A composition for use in the treatment of an infection of SARS-CoV-2 by targeting cannabinoid receptors, the composition comprising:
an emulsion formed from a pharmaceutically acceptable carrier mixed with active ingredients, wherein the pharmaceutically acceptable carrier is between 15-85 wt% of the composition, and wherein the active ingredients comprise:
a hemp extract, to provide a source of exogenous cannabinoids, in an amount of 5-40 wt% of the composition;
a cannabinoid enhancer comprising oleamide, to inhibit cannabinoid hydrolases, in an amount of 1.5-6 wt% of the composition;
a fatty acid amide, to enhance cannabinoid activity via an entourage effect, in an amount of 1.5-11 wt% of the composition;
a kava extract, to alleviate anxiety, in an amount of 6.0-35 wt% of the composition; and
an alkaloid, to enhance bioavailability of one or more of the active ingredients, in an amount of 0.2-3 wt% of the composition.

2. The composition for the use of claim 1, wherein:
the pharmaceutically acceptable carrier is a medium-chain triglyceride;
the fatty acid amide is palmitoylethanolamide (PEA); and
the alkaloid is piperine.

3. The composition for the use of claim 1, wherein at least some of the active ingredients are at least partially encapsulated with lecithin, and wherein the lecithin is present in an amount of 0.2-3 wt% of the composition, wherein the at least some of the active ingredients optionally includes the alkaloid.

4. The composition for the use of claim 1, wherein the hemp extract comprises at least one of cannabidiol (CBD), tetrahydrocannabinol (THC), cannabigerol, cannabinol, terpenes.

5. The composition for the use of claim 4, wherein the CBD is 99.5% of cannabinoids in the hemp extract.

6. The composition for the use of claim 4, wherein the hemp extract comprises CBD, and wherein the CBD is one of a full-spectrum CBD or a CBD isolate.

7. The composition for the use of claim 6, wherein the CBD is the CBD isolate, and wherein the effective amount of the hemp extract is 8 wt%.

8. The composition for the use of claim 6, wherein the hemp extract comprises beta-caryphyllene in an amount between 0.005-0.03 wt% of the composition.

9. A method of preparing a composition for treating an infection of SARS-CoV-2, the method comprising the steps of:
combining a pharmaceutically acceptable carrier with active ingredients to form a solution, wherein the active ingredients include:
a hemp extract, to provide a source of exogenous cannabinoids, in an amount of 5-40 wt% of the composition;
the cannabinoid enhancer oleamide, to inhibit cannabinoid hydrolases, in an amount of 1.5-6 wt% of the composition,
a fatty acid amide, to enhance cannabinoid activity via an entourage effect, in an amount of 1.5-11 wt% of the composition, and
an alkaloid, to enhance bioavailability of one or more of the active ingredients, in an amount of 0.2-3 wt% of the composition;
cooling the solution to a temperature less than 60°C;
adding a kava extract to the cooled solution; and
further cooling the cooled solution to a temperature less than 0°C to form the composition.

10. The method of claim 9, wherein the step of combining the pharmaceutically acceptable carrier with active ingredients further comprises: heating the pharmaceutically acceptable carrier to a temperature of at least 80°C before combining the active ingredients with the pharmaceutically acceptable carrier; and optionally dissolving the cannabinoid enhancer into the first intermediate solution when a temperature of the first intermediate solution between 70°C and 80°C to form a second intermediate solution; and further optionally dissolving the alkaloid into the third intermediate solution when a temperature of the third intermediate solution is between 70°C and 85°C to form a fourth intermediate solution.

11. The method of claim 9, wherein the step of combining the pharmaceutically acceptable carrier with active ingredients further comprises: dissolving the lecithin into the pharmaceutically acceptable carrier when a temperature of the pharmaceutically acceptable carrier is between 80°C and 90°C to form a first intermediate solution; and optionally dissolving the fatty acid amide into the second intermediate solution when a temperature of the second intermediate solution is between 70°C and 80°C to form a third intermediate solution; and further optionally adding the hemp extract to the fourth intermediate solution when a temperature of the fourth intermediate solution is between 70°C and 85°C to form the solution.

12. The method of claim 10, further comprising emulsifying the solution.

13. The method of claim 11, wherein the further cooling step is carried out for between five hours and ten hours, and wherein adding the kava extract to the cooled solution further comprises emulsifying the cooled solution.

14. The method of claim 9, wherein the hemp extract is cannabidiol isolate, and wherein the method further comprises adding a beta-caryphyllene to the cooled solution.

## Patentansprüche

1. Zusammensetzung zur Verwendung bei der Behandlung einer Infektion von SARS-CoV-2 durch Anzielen von Cannabinoidrezeptoren, wobei die Zusammensetzung Folgendes umfasst:
eine Emulsion, die aus einem pharmazeutisch unbedenklichen Träger gebildet ist, der mit Wirkstoffen gemischt ist, wobei der pharmazeutisch unbedenkliche Träger zwischen 15-85 Gew.-% der Zusammensetzung beträgt und wobei die Wirkstoffe Folgendes umfassen:
einen Hanfextrakt, um eine Quelle exogener Cannabinoide bereitzustellen, in einer Menge von 5-40 Gew.-% der Zusammensetzung;
einen Cannabinoidverstärker, umfassend ein Ölsäureamid, um Cannabinoidhydrolasen zu inhibieren, in einer Menge von 1,5-6 Gew.-% der Zusammensetzung;
ein Fettsäureamid, um eine Cannabinoidaktivität über einen Entourage-Effekt zu verstärken, in einer Menge von 1,5-11 Gew.-% der Zusammensetzung;
einen Kava-Extrakt, um Angst zu lindern, in einer Menge von 6,0-35 Gew.-% der Zusammensetzung; und
ein Alkaloid, um eine Bioverfügbarkeit eines oder mehrerer der Wirkstoffe zu verstärken, in einer Menge von 0,2-3 Gew.-% der Zusammensetzung.

2. Zusammensetzung zur Verwendung nach Anspruch 1, wobei:
der pharmazeutisch unbedenkliche Träger ein mittelkettiges Triglycerid ist;
das Fettsäureamid ein Palmitoylethanolamid (PEA) ist; und
das Alkaloid ein Piperin ist.

3. Zusammensetzung zur Verwendung nach Anspruch 1, wobei mindestens einige der Wirkstoffe mindestens teilweise mit einem Lecithin eingekapselt sind und wobei das Lecithin in einer Menge von 0,2-3 Gew.-% der Zusammensetzung vorhanden ist, wobei die mindestens einigen der Wirkstoffe gegebenenfalls das Alkaloid beinhalten.

4. Zusammensetzung zur Verwendung nach Anspruch 1, wobei der Hanfextrakt mindestens eines von einem Cannabidiol (CBD), einem Tetrahydrocannabinol (THC), einem Cannabigerol, einem Cannabinol, Terpenen umfasst.

5. Zusammensetzung zur Verwendung nach Anspruch 4, wobei das CBD 99,5 % von Cannabinoiden in dem Hanfextrakt beträgt.

6. Zusammensetzung zur Verwendung nach Anspruch 4, wobei der Hanfextrakt CBD umfasst und wobei das CBD eines von einem CBD mit vollem Spektrum oder einem CBD-Isolat ist.

7. Zusammensetzung zur Verwendung nach Anspruch 6, wobei das CBD das CBD-Isolat ist und wobei die wirksame Menge des Hanfextrakts 8 Gew.-% beträgt.

8. Zusammensetzung zur Verwendung nach Anspruch 6, wobei der Hanfextrakt ein Beta-Caryophyllen in einer Menge zwischen 0,005-0,03 Gew.-% der Zusammensetzung umfasst.

9. Verfahren zum Herstellen einer Zusammensetzung zum Behandeln einer Infektion mit SARS-CoV-2, wobei das Verfahren die folgenden Schritte umfasst:
Kombinieren eines pharmazeutisch unbedenklichen Trägers mit Wirkstoffen, um eine Lösung zu bilden, wobei die Wirkstoffe Folgendes beinhalten:
einen Hanfextrakt, um eine Quelle exogener Cannabinoide bereitzustellen, in einer Menge von 5-40 Gew.-% der Zusammensetzung;
den Cannabinoidverstärker Ölsäureamid, um Cannabinoidhydrolasen zu inhibieren, in einer Menge von 1,5-6 Gew.-% der Zusammensetzung,
ein Fettsäureamid, um eine Cannabinoidaktivität über einen Entourage-Effekt zu verstärken, in einer Menge von 1,5-11 Gew.-% der Zusammensetzung und
ein Alkaloid, um eine Bioverfügbarkeit eines oder mehrerer der Wirkstoffe zu verstärken, in einer Menge von 0,2-3 Gew.-% der Zusammensetzung;
Kühlen der Lösung auf eine Temperatur von weniger als 60 °C;
Hinzufügen eines Kava-Extrakts zu der gekühlten Lösung; und
weiteres Kühlen der gekühlten Lösung auf eine Temperatur von weniger als 0 °C, um die Zusammensetzung zu bilden.

10. Verfahren nach Anspruch 9, wobei der Schritt des Kombinierens des pharmazeutisch unbedenklichen Trägers mit Wirkstoffen ferner Folgendes umfasst: Erwärmen des pharmazeutisch unbedenklichen Trägers auf eine Temperatur von mindestens 80 °C vor dem Kombinieren der Wirkstoffe mit dem pharmazeutisch unbedenklichen Träger; und gegebenenfalls Auflösen des Cannabinoidverstärkers in die erste Zwischenlösung, wenn eine Temperatur der ersten Zwischenlösung zwischen 70 °C und 80 °C beträgt, um eine zweite Zwischenlösung zu bilden; und ferner gegebenenfalls Auflösen des Alkaloids in die dritte Zwischenlösung, wenn eine Temperatur der dritten Zwischenlösung zwischen 70 °C und 85 °C beträgt, um eine vierte Zwischenlösung zu bilden.

11. Verfahren nach Anspruch 9, wobei der Schritt des Kombinierens des pharmazeutisch unbedenklichen Trägers mit Wirkstoffen ferner Folgendes umfasst: Auflösen des Lecithins in den pharmazeutisch unbedenklichen Träger, wenn eine Temperatur des pharmazeutisch unbedenklichen Trägers zwischen 80 °C und 90 °C beträgt, um eine erste Zwischenlösung zu bilden; und gegebenenfalls Auflösen des Fettsäureamids in die zweite Zwischenlösung, wenn eine Temperatur der zweiten Zwischenlösung zwischen 70 °C und 80 °C beträgt, um eine dritte Zwischenlösung zu bilden; und ferner gegebenenfalls Hinzufügen des Hanfextrakts zu der vierten Zwischenlösung, wenn eine Temperatur der vierten Zwischenlösung zwischen 70 °C und 85 °C beträgt, um die Lösung zu bilden.

12. Verfahren nach Anspruch 10, ferner umfassend Emulgieren der Lösung.

13. Verfahren nach Anspruch 11, wobei der weitere Kühlschritt zwischen fünf Stunden und zehn Stunden lang ausgeführt wird und wobei das Hinzufügen des Kava-Extrakts zu der gekühlten Lösung ferner Emulgieren der gekühlten Lösung umfasst.

14. Verfahren nach Anspruch 9, wobei der Hanfextrakt ein Cannabidiol-Isolat ist und wobei das Verfahren ferner Hinzufügen eines Beta-Caryophyllens zu der gekühlten Lösung umfasst.

## Revendications

1. Composition pour utilisation dans le traitement d'une infection par le SARS-CoV-2 en ciblant les récepteurs cannabinoïdes, la composition comprenant :
une émulsion formée à partir d'un véhicule pharmaceutiquement acceptable mélangé à des principes actifs, dans laquelle le véhicule pharmaceutiquement acceptable représente entre 15 et 85 % en poids de la composition, et dans laquelle les principes actifs comprennent :
un extrait de chanvre, destiné à fournir une source de cannabinoïdes exogènes, en une quantité de 5 à 40 % en poids de la composition ;
un amplificateur de cannabinoïdes comprenant de l'oléamide, destiné à inhiber les hydrolases à cannabinoïdes, en une quantité de 1,5 à 6 % en poids de la composition ;
un amide d'acide gras, destiné à améliorer l'activité des cannabinoïdes par l'intermédiaire d'un effet d'entourage, en une quantité de 1,5 à 11 % en poids de la composition ;
un extrait de kava, destiné à soulager l'anxiété, en une quantité de 6,0 à 35 % en poids de la composition ; et
un alcaloïde, destiné à améliorer la biodisponibilité d'un ou de plusieurs des principes actifs, en une quantité de 0,2 à 3 % en poids de la composition.

2. Composition pour utilisation selon la revendication 1, dans laquelle :
le véhicule pharmaceutiquement acceptable est un triglycéride à chaîne moyenne ;
l'amide d'acide gras est le palmitoyléthanolamide (PEA) ; et
l'alcaloïde est la pipérine.

3. Composition pour utilisation selon la revendication 1, dans laquelle au moins une partie des principes actifs est au moins partiellement encapsulée avec de la lécithine, et dans laquelle la lécithine est présente en une quantité de 0,2 à 3 % en poids de la composition, dans laquelle ladite au moins une partie des principes actifs comprend éventuellement l'alcaloïde.

4. Composition pour utilisation selon la revendication 1, dans laquelle l'extrait de chanvre comprend au moins un parmi le cannabidiol (CBD), le tétrahydrocannabinol (THC), le cannabigérol, le cannabinol et des terpènes.

5. Composition pour utilisation selon la revendication 4, dans laquelle le CBD représente 99,5 % des cannabinoïdes dans l'extrait de chanvre.

6. Composition pour utilisation selon la revendication 4, dans laquelle l'extrait de chanvre comprend du CBD, et dans laquelle le CBD est soit un CBD à spectre complet, soit un isolat de CBD.

7. Composition pour utilisation selon la revendication 6, dans laquelle le CBD est un isolat de CBD, et dans laquelle la quantité efficace de l'extrait de chanvre est de 8 % en poids.

8. Composition pour utilisation selon la revendication 6, dans laquelle l'extrait de chanvre comprend du bêta-caryophyllène en une quantité comprise entre 0,005 et 0,03 % en poids de la composition.

9. Procédé de préparation d'une composition destinée au traitement d'une infection par le SARS-CoV-2, le procédé comprenant les étapes consistant à :
combiner un véhicule pharmaceutiquement acceptable avec des principes actifs pour former une solution, dans lequel les principes actifs comprennent :
un extrait de chanvre, destiné à fournir une source de cannabinoïdes exogènes, en une quantité de 5 à 40 % en poids de la composition ;
l'oléamide en tant qu'amplificateur de cannabinoïdes, destiné à inhiber les hydrolases à cannabinoïdes, en une quantité de 1,5 à 6 % en poids de la composition,
un amide d'acide gras, destiné à améliorer l'activité des cannabinoïdes par l'intermédiaire d'un effet d'entourage, en une quantité de 1,5 à 11 % en poids de la composition, et
un alcaloïde, destiné à améliorer la biodisponibilité d'un ou de plusieurs des principes actifs, en une quantité de 0,2 à 3 % en poids de la composition ;
refroidir la solution à une température inférieure à 60 °C ;
ajouter un extrait de kava à la solution refroidie ; et
refroidir de nouveau la solution refroidie à une température inférieure à 0 °C pour former la composition.

10. Procédé selon la revendication 9, dans lequel l'étape de combinaison du véhicule pharmaceutiquement acceptable avec des principes actifs comprend en outre : le chauffage du véhicule pharmaceutiquement acceptable à une température d'au moins 80 °C avant de combiner les principes actifs avec le véhicule pharmaceutiquement acceptable ; et éventuellement la dissolution de l'amplificateur de cannabinoïdes dans la première solution intermédiaire lorsque la température de la première solution intermédiaire est comprise entre 70 °C et 80 °C pour former une deuxième solution intermédiaire ; et en outre éventuellement la dissolution de l'alcaloïde dans la troisième solution intermédiaire lorsque la température de la troisième solution intermédiaire est comprise entre 70 °C et 85 °C pour former une quatrième solution intermédiaire.

11. Procédé selon la revendication 9, dans lequel l'étape de combinaison du véhicule pharmaceutiquement acceptable avec des principes actifs comprend en outre : la dissolution de la lécithine dans le véhicule pharmaceutiquement acceptable lorsque la température du véhicule pharmaceutiquement acceptable est comprise entre 80 °C et 90 °C pour former une première solution intermédiaire ; et éventuellement la dissolution de l'amide d'acide gras dans la deuxième solution intermédiaire lorsque la température de la deuxième solution intermédiaire est comprise entre 70 °C et 80 °C pour former une troisième solution intermédiaire ; et en outre éventuellement l'ajout de l'extrait de chanvre à la quatrième solution intermédiaire lorsque la température de la quatrième solution intermédiaire est comprise entre 70 °C et 85 °C pour former la solution.

12. Procédé selon la revendication 10, comprenant en outre l'émulsification de la solution.

13. Procédé selon la revendication 11, dans lequel l'étape de refroidissement supplémentaire est effectuée pendant une durée comprise entre cinq heures et dix heures, et dans lequel l'ajout de l'extrait de kava à la solution refroidie comprend en outre l'émulsification de la solution refroidie.

14. Procédé selon la revendication 9, dans lequel l'extrait de chanvre est un isolat de cannabidiol, et dans lequel le procédé comprend en outre l'ajout de bêta-caryophyllène à la solution refroidie.
